(19) Europäisches Patentamt
European Patent Office
Office européen des brevets

(11) Publication number: 0 130 166
A1

# (12) EUROPEAN PATENT APPLICATION

(21) Application number: 84850197.9

(51) Int. Cl.⁴: C 12 N 15/00

(22) Date of filing: 20.06.84

(30) Priority: 23.06.83 SE 8303626

(43) Date of publication of application:
02.01.85 Bulletin 85/1

(84) Designated Contracting States:
AT BE CH DE FR GB IT LI LU NL SE

(71) Applicant: KabiGen AB
S-112 87 Stockholm(SE)

(72) Inventor: Elmblad, Annette
Angermannagatan 199
S-16 222 Vällingby(SE)

(72) Inventor: Palm, Gunnar
Orvar Odds väg 5
S-112 54 Stockholm(SE)

(72) Inventor: Josephson, Staffan
Högstigen 2
S-182 74 Stocksund(SE)

(72) Inventor: Heden, Lars-Olof
Bygårdsvägen 25
S-185 00 Vaxholm(SE)

(72) Inventor: Holmgren, Erik
Lomovägen 3
S-181 47 Lidingö(SE)

(74) Representative: Burman, Tore et al,
Bergling & Sundbergh AB P.O. Box 7645
S-103 94 Stockholm(SE)

## (54) A recombinant plasmid, a transformant microorganism, a polydeoxy-ribonucleotide segment, a process for producing a biologically active protein, and the protein thus produced.

(57) A recombinant plasmid for use in transformation of a microbial host, said plasmid comprising a vector having colinearly inserted therein a synthetic production gene which codes for the expression of human insulin-like growth factor-I (IGF-I);

A transformant microorganism including a recombinant plasmid, said plasmid comprising a vector having inserted therein a synthetic production gene which codes for the expression of human insulin-like growth factor-I (IGF-I);

A polydeoxyribonucleotide segment which codes for the expression of human insulin-like growth factor-I (IGF-I);

A process for producing human insulin-like growth factor-I protein (IGF-I) which comprises culturing a transformant microorganism including a recombriant plasmid, said plasmid comprising a synthetic production gene which codes for the expression of human insulin-like growth factor-I (IGF-I), in a suitable nutrient medium until the protein is formed, and then isolating the said protein; and

Proteins obtained by said process.

EP 0 130 166 A1

./...

A1 A2 A3 A4 A5

5' GGAATTCT ATGGGTCCCGAAAC TCTGTGCGGTGCTG AACTGGTTGACGCT CTGCAGTTTGTTTG CGGTGA

3' CCTTAAGATACCCA GGGCTTTGAGACAC GCCACGACTTGACC AACTGCGAGACGTC AAACAAACGCCACT

B17 B16 B15 B14 B13

A6 A7 A8 A9 A10 A11

CCGTGGTT TTTATTTTAACAAA CCCACTGGTTATGG TTCTTCTTCTCGTC GTGCTCCCCAGACT GGTATTG

GGCACCAAAAATAA AATTGTTTGGGTGA CCAATACCAAGAAG AAGAGCAGCACGAG GGGTCTGACCATAA

B12 B11 B10 B9 B8

A12 A13 A14 A15

TTGACGA ATGCTGCTTTCGTT CTTGCGACCTGCGT CGTCTGGAAATGTA TTGCGCTCCCCTGA AACCCG

CAACTGCTTACGAC GAAAGCAAGAACGC TGGACGCAGCAGAC CTTTACATAACGCG AGGGGACTTTGGGC

B7 B6 B5 B4 B3

A16 A17

CTAAATCT GCTTAGAAGCTTGG 3'

GATTTAGACGAATC TTCGAACC 5'

B2 B1

Fig. 2

The two DNA-strands (for the IGF-1 gene) divided into oligomers A1-A17 and B1-B17. Besides the DNA code described in Fig. 1 the sequence has been provided with a start codon (ATG - in block A2), a stop codon (TAG - in block A17) and sequences for EcoRI (block A1) and HindIII (block A17).

A RECOMBINANT PLASMID, A TRANSFORMANT MICROORGANISM, A POLYDEOXYRIBONUCLEOTIDE SEGMENT, A PROCESS FOR PRODUCING A BIOLOGICALLY ACTIVE PROTEIN, AND THE PROTEIN THUS PRODUCED.

The present invention relates to a recombinant plasmid for use in transformation of a microbial host, to a transformant microorganism including such recombinant plasmid, and polydeoxyribonucleotide segment which codes for the expression of a special biologically active protein, namely the human insulin-like growth factor-I (IGF-I). The invention also covers a process for producing such protein, and the protein per se.

IGF-I is a polypeptide hormone (protein) closely related to proinsulin (see, for example, Rinderknecht et al. (1978), J.Biol.Chem., vol. 253, pp. 2769-2776 and Rinderknecht et al. (1978), FEBS Lett., vol. 89, pp. 283-286). It shows somatomedin activity in vitro (see, for example, Zapf et al. (1981), J.Clin.Invest. vol. 68, pp. 1321-1330 and Zapf et al. (1978), Eur.J.Biochem., vol. 87, pp. 285-296). Recent in vivo studies demonstrate stimulation of growth by IGF-I of hypophysectomized rats in a dose-dependent manner (Schoenle et al. (1982), Nature, vol. 296, pp. 252-253). The site of biosynthesis and storage of IGF-I is not known and the level of the protein in serum is very low (see, for example, Zingg et al. (1973), Diabetologia, vol. 9, p. 472).

The present invention has for its object to provide an easily accessible source of the polypeptide hormone in question.

Another object of the invention is to provide for synthesis of a production gene coding for the expression of human insulin-like growth factor-I.

Yet another object of the invention is to provide a recombinant plasmid containing a synthetic production gene, whereby in a microbial host the said plasmid can synthesise the mature protein. In this manner the said protein can be synthesised in significant quantities enabling production of antibodies and enabling biological studies of the insulin--like growth factor-I and also different biological uses

thereof.

In order to attain the said objects and further objects which will be clear from the following disclosure the invention provides a recombinant plasmid for use in transformation of a microbial host, the said plasmid comprising a vector having colinearly inserted therein a synthetic production gene coding for the expression of human insulin-like growth factor-I (IFG-I).

Such plasmid suitably enables translation in the correct phase for the mRNA corresponding to the inserted gene and having a bacterial promoter upstream of and adjacent to the insertion side so that the inserted gene is under bacterial promoter control.

It is preferred that plasmid used is derived from naturally occurring autogenerating genomes in procaryotic organisms. The gene is suitably provided with a start codon (ATG), a stop codon (TAG) and restriction enzyme sites. The codons of the gene are preferably trinucleotide codons.

Although it is to be noted that the invention is not limited thereto for the purposes of description the invention will be described with reference to a microorganism selected from the genus Escherichia, and more particularly the source of the plasmid vector used in the description is E.coli plasmid pBR 322 (described by Bolivar et al. Gene, 2, p.95, 1977).

The invention also provides for a transformant microorganism including a recombinant plasmid, the plasmid comprising a vector having inserted therein a synthetic production gene which codes for the expression of insulin-like growth factor-I (IGF-I). As a suitable host organism there may be used one selected fom the genus Escherichia, such as E.coli. However, it is to be understood that the invention is not limited to the use of solely such microorganisms since other organisms can be used as well, such as organisms selected from the genus Saccharomyces, Bacillus Neurospora, or Streptomyces, among others. For the purpose of illustrating the present invention there has been used as a host organism the microorganism E.coli K12, strains JM83 and 294.

The invention furthermore provides a polydeoxyribonucleotide segment coding for the expression of human insulin--like growth factor-I (IGF-I). Such segment is suitably provided with restriction endonuclease cleavage sites at the termini thereof, a start codon (ATG) inside one of said sites and a stop codon inside the other one of said sites, and a gene coding for the expression of insulin-like growth factor--I (IGF-I) between said codons. A preferred polydeoxyribonucleide segment has a structure essentially corresponding to that shown in Fig. 2 of the accompanying drawings.

According to another aspect of the invention there is provided a process for producing insulin-like growth factor-I protein, said process comprising culturing a transformant microorganism including a recombinant plasmid, said plasmid comprising a synthetic production gene which codes for human insulin-like growth factor-I (IGF-I), in a suitable nutrient medium until the protein is formed, and then isolating the said protein.

The invention also covers within its scope proteins prepared by the said process, particularly methionyl-IGF-I.

The invention will now be further illustrated by non--limiting examples with reference to the appended drawings in which:

Fig. 1 describes the particular preferred sequence selected for the coding portion of the synthetic IGF-I gene;

Fig. 2 is a representation of the synthetic oligonucleotide blocks constituting the synthetic gene;

Fig. 3 is a partial restriction enzyme map for the IGF-I-gene;

Fig. 4 is an illustration of the build-up of a completely protected tetradecanucleotide;

Fig. 5 is an explanation of symbols used below;

Fig. 6 is an illustration of the complete syntesized gene;

Fig. 7 illustrates construction of a chimeric cloning and expression vector;

Fig. 8 illustrates construction of a chimeric plasmid

containing the IGF-I gene;

Fig. 9 illustrates construction of another chimeric plasmid containing the IGF-I gene.

Fig. 10 illustrates changes both on the DNA and the protein level of the original IGF-I gene;

Fig. 11 illustrates how the point mutations were achieved on the IGF-I gene;

Fig. 12 illustrates colony hybridigation with the probes used;

Fig. 13 illustrates the cloning procedure for the IGF-I gene;

Fig. 14 shows the fused protein.

Although in the following examples the invention will be illustrated using E.coli as a host organism it is to be understood that the invention is not limited to the use of such organism but other organisms can be used as well.

IGF-I is known to consist of 70 amino acids with a sequence shown in Fig. 1 of the accompanying drawings (see, for example, Rinderknecht et al. (1978), J.Biol.Chem., vol. 253, pp. 2769-2776).

From the amino acid sequence in Fig. 1, a corresponding synthetic gene sequence has been invented, subject to a number of specific non-obvious criteria, and oligonucleotide blocks synthesised which, when assembled, form a synthetic gene coding for IGF-I. The blocks have been hybridized and ligated in pre-determined stages to construct the IGF-I gene. The synthetic gene has been provided with a start codon (ATG), a stop codon (TAG), and suitable restriction enzyme sites. This gene has been cloned into a specifically designed E.coli vector so as to produce a full length IGF-I gene flanked only by E.coli plasmid DNA. The gene has been excised from this recombinant and re-cloned into vectors specifically designed to maximise expression of the gene in E.coli, under the control of the promoter obtained from the E.coli lactose operon. A protein resembling IGF-I has thus been expressed in E.coli.

In order to construct nucleotide sequences which would

code for the desired protein several non-obvious criteria have been observed. Firstly, trinucleotide codons should be used which are acceptable or preferable in the cells to be used, in particular E.coli (see, for example, Wain-Hobson et al. (1981), Gene, vol. 13, pp. 199-209). Secondly, it was decided that it was desirable to have different restriction enzyme recognition sites at the termini of the molecule so as to allow inserteion into a plasmid in a desired orientation. Moreover, it was decided to select sites which allowed the use of well-understood cloning vectors, such as pBR322 (see, for example, Bolivar et al., (1977), Gene, vol. 2, pp. 95-113). In fact, EcoRI and HindIII sites were selected and introduced at the 5' and 3' ends- respectively. Thirdly, it was thought desirable to introduce a series of restriction endonuclease recognition sitesstrategically praced along the molecule to enable the gene to be specifically dissected to aid characterisation and, possibly mutagenesis. Fourthly, the synthesis should not be unnecessarily complicated and illegitimate crosshybridisations should be minimised in order to facilitate gene assembly.

In fact, bearing in mind, inter alia, the above mentioned considerations, for convenience, a slightly longer sequence was selected which is as described in Fig. 2 of the accompanying drawings and the restriction enzyme recognition sites are shown in Fig. 3 of the accompanying drawings.

In accord with this invention we have synthesised a molecule having the above expanded sequence by making 34 synthetic oligonucleotide blocks as illustrated in Fig. 2 of the accompanying drawings, which will assemble by single--strand overlaps to give the complete double-stranded nucleotide sequence.

Example I

Synthesis of tetradecanucleotide

As mentioned above, the synthetic blocks selected are shown in Fig. 2 of the accompanying drawings. The blocks are constructed using a fully automatic synthesising machine and

known syntheses technique (see, for example, Elmblad et al. (1982), Nucleic Acids Research, vol. 10, pp. 3291-3301 and Chow et al. (1981), Nucleic Acids Research, vol. 9, pp. 2807 -2817).

The synthetic methods will now be illustrated with reference to the synthesis of the tetradecanucleotide ApTpGpGpGpTpCpCpCpGpApApApC. (Fig. 2; A2).

The methods of building up oligonucleotides from individual nucleosidephosphites by successive coupling reactions are well known (see, for example, Elmblad et al. (1982), Nucleic Acids Research, vol. 10, pp. 3291-3301 and Chow et al. (1981). Nucleic Acids Research, vol. 9, pp. 2807-2817). The completely protected tetradecanucleotide was built up as shown in Fig. 4 of the accompanying drawings.

The coupling reactions were carried out by the following procedure exemplified by the synthesis of

DMTr $A^{Bz}$ $pC^{Bz}$-SILICA
OMe

(for explanation of symbols see Fig. 5 of the accompanying drawings).

DMTr-C-silica (100 mg 5 μmol) was transferred onto a column and detritylated with a solution of saturated $ZnBr_2$ in nitromethane (containing 1% water) followed by washing with dry Tetrahydrofurane (THF). To the detritylated C-silica there was added 5'-O-dimethoxytrityl-N-benzoyl-2'-deoxyadenosine-3-O-methyl phosphochloridite (50 μmol) in THF and reacted for 10 minutes at room temperature. The silica was again washed with THF and the phosphite oxidized with iodine followed by another THF wash. The last step in the cycle was acetylation of non-reacted C-silica. The cycle was repeated the required number of times to obtain the tetradecanucleotide. The oligomers were deprotected firstly by treatment with thiophenol-triethylamine in dioxane for 30 minutes at room temperature which removes the methyl protecting group of the internucleotide phosphotriester, and then with concentrated ammonia at 50°C for 4 hours. The silica

was removed by filtration and the ammonia solution evaporated. The DMTr-tetradecamers were redissolved in water and purified by high-pressure-liquid-chromatography (HPLC) on an Ultracil reversed phase C18 5μ column, Altex HPLC, detritylated with acetic acid (80%) 15 minutes at room temperature and injected again on the HPLC using a Spherisorb® reversed phase C18 5μ column LDC-HPLC-system.

The oligomeric blocks of nucleotides were hybridised and ligated (cf. Khorana et al., (1976), J.Biol.chem., vol. 251, pp. 634-675) in a series of steps, in order to minimise the possibilities for undesirable interactions, leading to the formation of the complete gene as shown in Fig. 6 of the accompanying drawings. The order of the additions in the assembly scheme was optimised for minimal incorrect ligations.

Ligation of block I

Oligomers A2 (14 μl 1 nmol in water), B17 (14 μl 1 nmol in water), and B16 (14 μl 1 nmol in water) were each mixed with 5 μl 10xLigation buffer (0.5 M Tris-HCl, pH 7.7, 0.1 M $MgCl_2$), 25 μl water, 1 μl 0.5 M dithiothreitol (DTT), T4 polynucleotide kinase (kinase) (2 μl, 2 units/μl; one unit of enzyme is defined as that amount catalyzing the transfer of 1 nmol of phosphate from ATP to 5'-OH termini of RNA or DNA in 30 minutes at 37°C) and $^{32}P$-γATP (3 μl 11 pmol specific activity 2900.0 Ci/mmol). The mixtures were incubated at 37°C for 10 minutes, then unlabelled ATP (2 μl 1.0 mM) was added together with another 2 units of kinase and the mixtures were incubated for another hour at 37°C. The oligomers were checked on a short 20% denaturing polyacrylamide gel (7 M urea in 50 mM Tris-borate, pH 8.3/0.8 mM EDTA).

The three phosphorylated oligomers were mixed without further purification and A1 (14 μl 1 nmol in water) was added together with 2 μl 10xLigation buffer. The mixture was heated at 80°C for 5 minutes and cooled to 10°C during 2 hours. To the cooled solution there was added DTT (1 μl 0.5 M), ATP (2 μl 1.0 mM) and DNA-ligase (T4) (2 μl 20 Weiss units; one unit of ligase is defined as the amount of enzyme

that catalyzes the exchange of 1 nmol of phosphate from ATP into pyrophosphate in 20 minutes at 37°C). Ligation was performed at 4°C overnight. The sample was phenol and ether extracted followed by ethanol precipitation by the addition of 2.5 volumes of 99.5% ethanol. The ligation product was recovered by centrifugation for 10 minutes at 9,980xg and finally purified on a 30 cm long 1.5 mm thick 15% nondenaturing polyacrylamide gel which was run at 200 V for 16 hours with cooling to +4°C. This gives a distinct band for the product which was detected both by autoradiography and visibility under UV-light at 254 nm. The DNA fragment was eluted from the gelpiece with ammonium acetate (300 µl 3 M) over night at room temperature. The ammonium acetate solution was collected, diluted with water to a final concentration of 0.05 M. The sample was then applied to a DE-52 ion exchange column and the DNA was eluted by 3 M ammonium acetate. The solution was lyophilized and the yield determined by measuring the absorbance at 260 nm.

Ligitation of blocks III, V, VI, and VII

Ligation of these blocks were done exactly as for block I.

Example II

Ligation of block II

Ligation of block II was done exactly as for block I until purification. Purification was instead done on a 20% denaturing polyacrylamide gel. The two main bands, the 42-mer from the A-chain and the 28-mer from the B-chain were cut out of the gel, eluted with ammonium acetate (300 µl 3 M) run through a DE-52 ion exchange column and evaporated. The two fragments were separately phosphorylated in the same way as described above. After phosphorylation, the two oligomers were mixed and heated at 80°C or 5 minutes and cooled to 10°C over a period of 2 hours. Block II now formed was purified on a 15% nondenaturing polyacrylamide gel. The band was eluted as described above.

Example 111

Ligation of block IV

Block IV was ligated in two steps. First 1 nmol each ofA9 and B10 (see Fig. 2) were phosphorylated as described for block 1, and then mixed with 1 nmol A8 and 1 nmol B9 (see Fig. 2) and ligated as for block 1 followed by purification. The pure fragment was phosphorylated and mixed with 2 nmol phosphorylated A10 and 2 nmol phosphorylated B8 (see Fig. 2) and ligated as above. This mixture gave, when separated on a 15% nondenaturing polyacrylamide gel, two bands, one being the desired block IV and the other being the ligation product of A10/B8 ligated to itself. Block IV was purified as block I.

Example IV

Ligation of block I and II to form block a

Block I (100 pmol in 20 µl) and block II (60 pmol in 12 µl) were mixed together with 4 µl 10xLigation buffer and 1 µl water. This solution was heated at 50°C for 5 minutes and cooled to 10°C over a period of 2 hours. Then ATP (2 µl 1 mM), 1 µl 0.5 M DTT and 2 µl ligase (10 units/µl) was added and the mixture was ligated at 4°C for 2 hours. The ligation mixture was then phenol and ether extracted and precipitated with ethanol as described above. The precipitated material was finally purified on a 15% nondenaturing polyacrylamide gel. The band (block a) was eluted from the gel and purified in the same way as for block I.

Example V

Ligation of block III and IV to form block b and VI and VII to form block d

After phosporylation of block III (150 pmol) it was mixed with block IV (100 pmol) and they were ligated and purified in the same way as for block a. After phosphorylation of block VI (170 pmol) it was mixed with block VII (200 pmol), this mixture was also ligated and purified in the same way as block a.

Example VI

Ligation of block a and b to form Block A and V and d to form block B

20 µl (70 pmol) phosphorylated block b and 10 µl (50 pmol) block a were mixed and 1 µl 1 mM ATP, 4 µl 10xLigation buffer, and 4 µl water was added. The mixture was heated at 50°C for 5 minutes and cooled to 10°C over a period of 2 hours. To this mixture was then added 1 µl 0.5 M DTT and 2 µl ligase (10 units/µl) and the mixture was ligated at 4°C for 2 hours and then purified as described for block a above, giving block A. Block V (100 pmol) was phosphorylated and ligated to d (100 pmol) to give block B exactly in the same way as for block A.

Example VII

Ligation of block A and B to give the IGF-I gene

Block A (5 pmol in 2 µl) was mixed with block B (10 pmol in 2 µl). To this solution were added 2 µl 10xLigation buffer, 5 µl 50 µM ATP, and 10 µl water. The mixture was heated at 50°C for 5 minutes and cooled to 10°C over a period of 2 hours. To the mixture were then added 1 µl 0.5 M DTT and 1 µl T4-ligase (10 units/µl) and the mixture was ligated at 4°C for 2 hours followed by phenol and ether extraction, and finally precipitated with ethanol as described above.

0.5 pmol of the complete ligation product in 50 mM Tris-HCl, pH 7.6/10 mM $MgCl_2$/100 mM NaCl was digested with the restriction enzymes EcoRI and HindIII (40 units of each; one unit is defined as the amount of enzyme required to produce a complete digest of 1.0 µg DNA in 60 minutes in a total volute of 0.05 ml) for 60 minutes at 37°C. The mixture was extracted with phenol and ether and separated on a nondenaturing 10% polyacrylamide gel in 89 mM Tris-HCl, pH 8.3/89 mM boric acid /2.5 mM $Na_2EDTA$. The fragment running at a position of about 230 base pairs (bp) was electroeluted as described by H.O. Smith in Methods in Enzymology (1980) vol. 65, pp. 371-380. The electroeluted material was phenol extracted and extracted extensively with ether to eliminate

any trace of phenol. This material was used for the cloning of the IGF-1 gene in a vector as described below.

A particular vector, designated pKG1, has been specifically designed to afford advantages for the purposes of the present invention. The inventive plasmid, pKG1, is a 4.6K base pair plasmid which can be propagated in E.coli and which may be constructed from pBR322 (see Bolivar et al. (1977), Gene, vol. 2, pp. 95-113) and pKB258 (see Backman et al. (1978), Cell, vol. 13, pp. 65-71) by inserting the 285 bp EcoRI fragment of pKB258 into the EcoRI site of pBR322 in such a way that the transcription from the lactose promoter is directed towards the tetracycline resistance gene of pBR322 and deletion of the EcoRI restriction enzyme site upstream of the lactose promoter region.

Example VIII

Construction of the chimeric cloning and expresseion vector pKG1

In order to facilitate the expression of IGF-1 in E.coli, it was desirable to construct a vector or vectors having a strong promoter and suitable restriction enzyme sites, see Fig. 7 of the accompanying drawings. 20 µg of the plasmid pHGH107 (see Goeddel et al., (1979), Nature, vol. 281, pp. 544-548) containing the promoter fragment referred to above was digested with the restriction enzymes EcoRI and BamHI (30 units of each enzyme) in the buffer described above at 37°C for 2 hours. The material was separated on agarose gel as described above and the large fragment, containing the promoter fragment was electroeluted as described above and extracted with phenol and ether and finally ethanol precipitated as described above. Similarly, 20 µg of the plasmid pBR322 (see Bolivar et al. (1977), Gene, vol. 2, pp 95-113) was digested with EcoRI and BamHI exactly as above and separated on an agarose gel. The smaller fragment, containing the part of the tetracycline resistance gene missing in the large fragment above, was electreluted, extracted and precipitated as above. Equimolar amounts of the fragments were mixed and 2 µl buffer (660 mM Tris-HCl, pH 7.5/100 mM $MgCl_2$/100 mM DTT),

2 µl 5 mM ATP, 2 units T4-ligase, and water to 20 µl was added. The mixture was incubated at 20°C for 2 hours. 10 µl of the ligation mixture was used to transform E.coli K12 strain 294 (endo I, B1, $r_k^-$, $m_k^+$) (see, for example, Dagert et al. (1979), Gene, vol. 6, pp. 23-28.) After 60 minutes for phenotypic expression the cells were plated on Luria agar plates (see Miller, Experiments in Molecular Genetics (Cold Spring Harbor, N.Y., 1972)) supplemented with 12.5 µg/ml of tetracycline, to which the plasmid confers resistance, for the selection of transformants, After overnight incubation at 37°C individual colonies were checked for the presence of the lactose promoter fragment by restriction enzyme digestion of plasmid DNA prepared according to known methods (see Birnboim et al. (1979), Nucleic Acids Research, vol. 7, pp. 1513-1532). One colony containing the expected promoter fragment was subjected to DNA sequence analysis to confirm the insertion of the lactose promoter. This plasmid was designated pKG1 (see Fig. 7 of the accompanying drawings).

Plasmid pKG1 using E.coli, strain 294 as a host cell has been deposited with Deutsche Sammlung von Mikroorganismen (DSM), Gesellschaft für Biotechnologische Forschung mbH, Göttingen, West Germany, under deposition Number DSM 2673.

Example IX

Construction of the chimeric plasmid pKG3

The plasmid pUC8 (see Vieira et al. (1982), Gene, vol. 19, pp. 259-268( was digested with the restriction enzymes EcoRI and HindIII as described above. The digested material was separated on a 0.8% agarose gel in 40 mM Tris-HCl, pH 8.1/20 mM acetic acid/2 mM $Na_2$EDTA and the large fragment (about 2700 bp) was electroeluted as described above. After extraction with phenol and ether the material was precipitated with ethanol as described above. The precipitated material was resuspended to a concentration of 0.1 pmol/µl in water 0.05 pmol of the IGF-I fragment (5 µl) from above was mixed with 0.5 pmol of the large pUC8 DNA fragment and the followin was added: 4 µl buffer (660 mM Tris-HCl, pH 7.5/100 mM

$MgCl_2$/100 mM DTT), 4 µl 1 mM ATP. The mixture was heated at 68°C for two minutes and was then slowly cooled to 4°C. 6 units T4 ligase were then added and the ligation mixture was incubated at 12°C for 6 hours. The bacterial strain JM83 (a a, Δ lac-pr o, strA, thi, Ø 80d lacZ Δ 1115, see see Vieira et al. (1982), Gene, vol. 19, pp. 259-268) was transformed with 10 µl of the ligation mixture as described above but with 50 µg/ml ampicillin instead of tetracycline. The transformants obtained after 18 hours incubation of the agar plates at 37°C were screened for the presence of the IGF-I gene by colony hybridisation as described by Gergen et al. (1979), Nucleic Acids Research, vol. 7, pp. 2115-2136 using kinated fragment B16 (see Fig. 2 of the accompanying drawings) as probe. The presence of a complete IGF-I gene in the colonies showing a positive hybridization signal was confirmed by restriction enzyme mapping taking advantage of the expected restriction enzyme sites given in Fig. 3 of the accompanying drawings and by nucleotide sequence analysis using well known techniques (see Sanger et al. (1977), Proc. Natl.Acad.Sci.USA, vol. 74, pp. 5463-5467). The plasmid containing the IGF-I gene obtained in this manner was designated pKG3 (see Fig. 8 of the accompanying drawings).

Example X

Construction of the chimeric plasmid pKG4 containing the IGF-I gene

Any IGF-I protein synthesised in bacterial cells containing the plasmid pKG3 will contain at its aminoterminal end 6 amino acids derived from the β-galactosidase since the IGF-I gene is fused to the gene for β-galactosidase (see, for example, Vieira et al. (1982), Gene vol. 19, pp. 259-268). To obtain a plasmid coding for an IGF-I without any extra amino acids, the IGF-I gene was recloned into pKG1. The recloning into pKG1 was performed in such a way that IGF-I synthesised in bacterial cells containing this modified plasmid would not contain any extra amino acids, except the methionine coded for by the start codon ATG. The

plasmid pKG1 was digested with EcoRI and HindIII as described above and the fragments were separated on an 0.8% agarose gel as described above and the large fragment was electroelute, phenol and ether extracted and finally ethanol precipitated as described above. Plasmid pKG3 was digested in exactly the same way and the small fragment (the IGF-I gene) was purified as above. 200 ng of the large fragment from pKG1 were mixed with an equimolar amount of the small fragment from pHG3. 10 µl buffer (660 mM Tris-HCl, pH 7.5/100 mM $MgCl_2$/100 mM DTT), 0.6 units T4-ligase, 10 µl 1 mM ATP, and water to 100 µl was then added. The mixture was incubated at 12°C over night and used for transformation of E.coli K12 strain JM83 with selection for tetracycline resistance as described above. The plasmid pKG1 was constructed in such a way that when the IGF-I gene was cloned into the EcoRI/HindIII sites tetracycline resistant transformant would only occur if the IGF-I gene is transcribed. Single colonies from the transformation occurring after 18 hours incubation at 37°C were checked for the presence of the IGF-I gene by preparing plasmid DNA and subjecting this to restriction enzyme analysis taking advantage of the restriction enzyme map given in Fig. 3 of the accompanying drawings. One transformant containing the expected fragment was designated pKG4 (see Fig. 9 of the accompanying drawings).

Example XI

Expression of IGF-I in E.coli

E.coli K12 strains JM83 and 294 containing the plasmids pKG3 and pKG4, respectively, were grown in Luria broth (LB) (see, for example, Miller, Experiments in Molecular Genetics (Cold Spring Harbor, N.Y., 1972)) with aeration at 37°C to an optical density of 1.0 at 600 nm. The cells were collected by centrifugation 10 minutes, 8000 rpm at 4°C. After washing the cells once in TE (10 mM Tris-HCl, pH 7.0/1 mM $Na_2$EDTA) the cells were resuspended in 1/10 of a volume of TE and subjected to sonication 10 times with bursts of 30 seconds with cooling in ice water in between bursts. Insoluble material

was eliminated by centrifugation as above and the supernatant was analyzed for the presence of IGF-I like material. 10 µl aliquotes of dilutions were applied to nitrocellulose filter paper and subjected to immunological analysis, using rabbit antibodies raised against purified IGF-I from human serum and the peroxidase-antiperoxidase method (see Glass, (1981), Science, vol. 211. pp. 70- ).

Cells of E.coli K12 strains JM 83 and 294 containing the plasmid pKG3 and pKG4 have been deposited with Deutsche Sammlung von Mikroorganismen (DSM), Gesellschaft für Biotechnologische Forschung mbH, Göttingen, West Germany, under deposition numbers DSM 2674 and 2675, respectively.

Production of IGF-1 in S. aureus via a fusion protein

In order to get a high production of IGF-1, it was produced as a fusion protein together with protein A. When working with fusion proteins one has to introduce cleavage sites between the desired protein and the fusion. This was done by changing the synthetic gene in the 5'-end and hereby introducing new cleavage sites on protein level. See Fig. 10. The changes of the synthetic gene was achieved by *in vitro* mutagenesis. Three different mutations were done.

1. The N-terminal glycine GGT was mutated to aspartic acid GAC see Fig 10 and 11. By doing this amino acid exchange a new aspartic acid proteine bond was formed. This allows for gene fusions encoding hybrid proteins that can be cleaved apart, before or after purification, by formic acid treatment whic cleaves between aspartic acid and proline (Landon, Methods in Enzymology 47 132-145, 1977). This gives IGF-1 lacking tł N-terminal glycine.

2. The extra methionine ATG in front of the gene was mutated t( asparagine AAC, see Fig. 10 and 11. By doing this amino aci exchange an aspargine glycine bond was formed which can be cleaved, before or after purification, by hydroxylamine (Landon, Methods in Enzymology 47 132-145 1977). This gives the native IFG-1 protein.

3. The same methionine ATG as above was mutated to a tryptopha TGG. By doing this amino acid exchange we introduce a clea\ site after the tryptophane. This cleavage is done by N-bror succinic imide (Landon, Methods in Enzymology 47 132-145 19 This method also gives a native IGF-1 protein.

As an example of the in vitro mutagenesis the Gly→Asp exchange is described.

Example XII

In vitro mutagenesis of the synthetic gene to encode a modified human IGF-1

10 µg of plasmid pKG3 was cleaved with Eco RI and Hind III and a 0.22 kb fragment thereof was cut out from a 5% polyacrylamide gel after electrophoresis. 50 ng of purified fragment was mixed with 200 ng of phage M13 mp9 and cleaved with Eco RI and Hind III in a total volume of 20 µl. After treatment with T4-ligase the DNA was used to transform E.coli JM83 and the cells were spread on AXI-plates. Cleavage, ligation and transformation were performed as described above. Phage purification from one white plaque was performed (Messing, Methods in Enzymolog 20-78 1983).Using the universal primer (Bio-Labs, New England, USA) the phage insert was confirmed to be the 220 bp synthetic IGF-1 gene. This phage, disignated mg9/IGF-1, was used for the following mutagenesis.

One primer oligonucleotide consisting of 24 bases (5'-GTGAATTCTATGGACCCCGAAACT-3') which was used for the mutagenesis and one probe (P) oligonucleotide consisting of 14 bases (5'-AATTCTATGGACCC-3') which was used to identify successfully mutagenized phage clones. The mismatches between the synthetic IFG-1 gene and the primer are shown in Fig. 11.

6 pmoles of mp9/IFG-1 and 80 pmoles of primer were mixed in a total volume of 80 ul containing 100 mM NaCl, 20 mM $MgCl_2$ and 40 mM TRIS-HCL, pH 7.5. The mixture was heated to 65°C for

3 minutes and allowed to cool to 23°C for 30 minutes. After transfer to an ice-bath, 190 µl of $H_2O$ and 30 µl of a solution containing 100 mM $MgCl_2$, 50 mM DTT and 200 mM TRIS-HCl, pH 7.5, was added. 50 units of Klenow fragment (Boehringher-Mannheim, West-Germany) was added and after 10 minutes in an ice-bath the sample was brought to 23°C for 30 minutes. Another 50 units of Klenow fragment was added and after 60 minutes at 23°C the polymerase was heat inactivated at 65°C for 10 minutes. The sample was precipitated once with ethanol, followed by cleavage with Eco RI and Hind III. The 0.22 kb fragment was cut out from a 5% polyacrylamide gel after electrophoresis and the fragment was eluted and purified as described above.

50 ng of purified fragment were mixed with 200 ng of phage M13 mp9 cleaved with Eco RI and Hind III in a total volume of 20 µl. After ligation and transformation to E.coli JM83, exactly as described above, white plaques were fou in a background of blue plaques. 48 white plaques were further analysed by hybridization with two synthetic probes as describe by Winter et al (Nature 299, 21 October, 1982). The filters were hybridized at room temperature with 32 P-labelled oligonucleotides and washed at different temperatures. Using probe A2, 5'-ATGGGTCCCGAAAC-3'

all clones except four show strong hybridization after wash at 44°C indicating that these clones contain tl original IGF-1 gene. Using probe P, 5'-AATTCTATGGACCC-3', all four of the previously negative clones showed significant hybr: dization. One of the four phages, designated mp9/IGF-1.M3, was further sezuenced using the universal primer as described abov. This confirmed a successful mutagenesis as shown in Fig. 12.

200 ng of phage mp9/IGF-1.M3 and 200 ng of plasmid pUC8 were separately cleaved with Eco RI and Hind III. After T4-ligase treatment, in a total volume of 20 µl, the DNA was used to tra form E.coli JM83 and the cells were plated out on AXI-plates.

Cleavage, ligation and transformation were performed as described. Restriction analysis of a white colony revealed the expected plasmid, pUC8, containing a 0.22 kb Eco RI/Hind III insert. This plasmid was designated pKG11 and was used for the following steps.

C. Construction of shuttle plasmid pUN200 containing pKG11

1 µg of pKG11 from step B and 2 ug of pH14, both digested with Hind III, were mixed and ligated in a total volume of 100 µl overnight at +14°C. After digestion with Eco RV, the DNA-mixture was transformed to E.coli HB101 and plated on LA-plates containing 50 ug ampicillin per milliliter. 52 single colonies were picked to LA plates containing 10 µg/ml of chloramphenicol and 50 µg/ml of ampicillin. After two days at 28°C one clone appeared and the plasmid in this clone was further characterized by restriction analysis. This revealed plasmid pUN200 schematically shown in Fig. 13. This plasmid, which contains the IGF-1 gene, can replicate both in E. coli and S. aureus.

D. Construction of shuttle plasmids pUN201 and pUN202

1 ug of pUN200 and 1 µg of plasmid pSPA16, both digested with Eco RI, were mixed and ligated in a total volume of 100 µl overnight at 14°C. The ligase was heat inactivated at 65°C for 10 minutes. After digestion with Eco RV to decrease the number of background clones containing pSPA16, the DNA mixture was transformed to E. coli HB101 and plated out on LA-plates containing 50 µg of ampicillin. Plasmids from 48 clones were analyzed by restriction mapping and 3 thereof were found to contain pUN200 with a 1.1 kb Eco RI insert from pSPA16, corresponding to the 5'-end of the protein A gene. The orientation of the insert in these three plasmids was further analyzed by cleavage with Hind III and two were found to contain a predicted fusion between the genes encodin

protein A and IGF-1. This plasmid was designated pUN201 (Fig. 13). The nucleotide sequence and the deduced amino acid sequence of this gene fusion are shown in Fig. 14. The predicted molecular weight of the mature hybrid protein is 38,701.

One of the three clones was found to contain a plasmid, designated pUN202, with opposite orientation of the protein A gene versus the IGF-1 gene (Fig. 13). This plasmid codes for a truncated protein A with a predicted molecular weitht of 30,963.

II. Transformation of shuttle plasmids pUN201 and pUN202 to S. aureus SA113

10 µg of plasmids pUN201 and pUN202 from step ID above were used to transform protoplasts of S. aureus SA113 as described by Götz, F. et al, J. Bacteril. 145, 74-81 (1981) and in the International patent application PCT/SE83/00297 (Step IIIA). Chloramphenicol resistent clones were found after 3 days at 37°C and these transformants were restreaked on TSA-plates (Trypticase Soy Agar) with chloramphenical (10 µg/ml). One transformant of the respective plasmid (pUN201 and pUN202) was chosen for further analysis. Restriction mapping of the purified plasmids revealed that the intact plasmid had been introduced into the S. aureus SA113 host.

III. Quantification and localization of the protein A activity from clones carrying pUN201 and pUN202

E. coli cells carrying pUN200, pUN201 and pUN202 respectively (from Step IC and D) above and S. aureus cells carrying pUN201 or pUN202 were cultivated in 200 ml of liquid medium overnight. E. coli strains were grown in LB medium with ampicillin (50 µg/m and S. aureus strains in TSB (Trypticase Soy Broth) with chloramphenicol (10 µg/ml). The cells were pelleted by centrifugation at 6000 rpm with a Sorwall GSA-rotor for 10 minutes and the supernatant, designated medium, was saved. The cell pellet was washed in 10 ml of PBS + TWEEN and again centrifuged as above. This time the cell pellet was resuspended in 10 ml of a protease inhibitor buffer (0.02 M potassium phosphate, pH 7.5, 0.1 M NaCl, 0.5% sodium deoxycholate, 1% Triton x-100, 0,1% sodiumdodecyl sulfate (SDS), and 1 mM phenylmethylsulfonyl fluoride (PMSF). The cells were then sonicated in a MSE sonicator for 4 x 40 sec. on an ice-bath and centrifuged at 15,000 rpm (Sorvall SS-34 rotor) for 10 min. The supernatant, designated cell extract, was collected and an ELISA-test was performed to determine the amount of protein A in the samples. The results are shown in the following Table 1.

**Table 1**

Amount of protein A per ml of sonicated cell culture determined by the ELISA-test. Zero-values correspond to less than 0.1 µg/ml.

| Host (Plasmid) | Cell extract (µg/ml) | Medium (µg/ml) |
|---|---|---|
| E. coli HB101 (pUN200) | 0 | 0 |
| E. coli HB101 (pUN201) | 2 | 0 |
| E. coli HB101 (pUN202) | 2 | 0 |
| S. aureus SA113 (pUN201) | 0.2 | 5 |
| S. aureus SA113 (pUN202) | 0 | 5 |
| E. coli HB101 | 0 | 0 |
| S. aureus SA113 | 0 | 0 |

Table 1 shows that in both E. coli and S. aureus the amount of protein A produced is not influenced by the orientation of the

fragment containing the protein A gene (plasmid pUN201 versus pUN202). Thus, the protein A IGF-1 hybrid protein encoded by pUN201 is produced at approximately the same level as the truncated protein A encoded by pUN202. Both proteins are, as expected, found in the cell extract of E. coli and in the medium of S. aureus.

IV. Purification of IGF-1 by IgG-affinity chromatography and formic acid treatment

The media of S. aureus SA113 carrying pUN201 and pUn202 respectively, from Step III, were each passed over an IgG-Sepharose$^R$ 4B column (Pharmacia AB, Uppsala, Sweden) (Hjelm et al, FEBS Lett. 28, 73-76 (1972)) that had been equilibrated with a sodium acetate buffer (0.1 M sodium acetate, 2% NaCl, pH 5.5). The column was then washed with the same buffer as above and the adsorbed protein A eluted with a glycine buffer (0.1 M glycine, 2% NaCl, pH 3.0). The eluted fraction was dialyzed against distilled water and thereafter lyophilized in two aliquots. The protein pellet of one of the aliquots was analysed on a 13% SDS-polyacrylamide gel at 100 V for 12 hours. The gel was stained with amidoblack (0.196, in 45% methanol, 10% acetic acid). This revealed a major protein with the molecular weight of 38,701 for pUN210 and 30,963 for pUN202. This is in accordance with the predicted sizes deduced from the DNA sequences (see Step ID above).

The second aliquot was resuspended in 0.5 ml of 70% formic acid and further incubated at 37°C for 2 days. This process cleaves proteins at the dipeptide sequence aspartic acid-proline. The predicted degradation products from the hybrid protein encoded by pUN201 were, apart from the IGF-1 moiety lacking the N-terminal glycine, five oligopeptides of the molecular weights of 6800, 6600, 6600, 6600 and 600. SDS-polyacrylamide electrophoresis, as described above, also confirms that the major prote

bands are shifted from approximately 38000 to several bands around 7000.

The formic acid treated proteins were lyophilized and resuspended in distilled water. The sample from pUN201 was passed over an IgG-Sepharose$^R$ 4B column as described above. The flow through and the eluted material (with the glycine buffer) were saved for further analysis.

V. Analysis of the protein products by radio receptor assay (RRA)

The radio receptor assay (RRA) was performed according to Hall et al, J. Clin. Endocrinol. Metab. 48, 271-278 (1974) using a particulate fraction of human placental membrane as matrix. The standard used in the assay consisted of pooled normal human serum having a potency of 1 unit (U) of IGF-1 per ml. The lower limit of the assay was 10 mU per ml. The peptide used for labelling was purified from Cohn fraction IV to a specific activity of 500 U/mg protein (according to RRA). Labelling of the peptide was performed according to Thorell et al. Biochem. Biophys. Acta 251, 363-369 (1971). Purification of the tracer was done on a carboxymethyl cellulose column using an elution gradient of 0.1 M $NH_4OAc$ from pH 4.0 to pH 6.8. The specific activity of the tracer was approximately 20 µCi/µg. The assay was performed as follows:

The standard or unknown sample (100 µl) was incubated together with 100 µl of placental membrane and 100 µl of labelled IGF-1 overnight at + 4°C. After centrifugation the pellet was washed once and counted in a gamma counter. The sample potency was calculated using an "in house" computer program.

Samples before and after the formic acid treatment, from Step IV above, were analyzed by the RRA-test and the results are shown in Table 2 below.

Table 2

Radio receptor analysis (RRA) for IGF-1 activity in growth medium from S. aureus SA113 (pUN202) and S. aureus SA113 (pUN201) after isolation and purification by IgG affinity chromatography. Zero corresponds to less than 1 U/l medium.

| Plasmid | Treatment | Activity (U/l) |
|---|---|---|
| pUN202 | Before treatment with formic acid | 0 |
| | After treatment with formic acid | 0 |
| pUN201 | Before treatment with formic acid | 0 |
| | After treatment with formic acid | 143 |
| | Flow through,[x] | 106 |
| | Eluate,[x] | 19 |

[x] A formic acid treated sample was passed over an IgG-Sepharose[R] column and the activity was measured for bound (eluate) and not bound (flow through) IGF-1.

From Table 2 it appears that the hybrid protein encoded by pUN201 has no detectable IGF-1 activity. Treatment with formic acid yields IGF-1 activity, and most of this activity does not bind to the IgG affinity column indicating a successful cleavage between the protein A and the IGF-1 moiety.

While embodiments of the invention have been presented above, the invention is, of course, not restricted thereto, but many variations and modifications are possible without departing from the scope thereof as defined by the susequent claims.

It is to be understood that the invention is not to be limited to the exact details of operation or exact genes, vectors, plasmids or procedures shown and described above, as obvious modifications and equivalents will be apparent to one skilled in the art, and the invention is therefore to be limited only by the full scope of the appended claims, including the application of the doctrin of equivalents thereto.

0130166

PATENT CLAIMS:

1. A recombinant plasmid for use in transformation of a microbial host, said plasmid comprising a vector having colinearly inserted therein a synthetic production gene which codes for the expression of human insulin-like growth factor -I(IGF-I).

2. A plasmid according to claim 1 enabling translation in the correct phase of the mRNA corresponding to the inserted gene and having a bacterial promoter upstream of and adjacent to the insertion site so that the inserted gene is under bacterial promoter control.

3. A plasmid according to claim 1 or 2 derived from naturally occurring autogenerating genomes in procaryotic organisms.

4. A plasmid according to any preceding claim, wherein said gene is provided with a start codon (ATG), a stop codon (TAG) and restriction enzyme sites.

5. A plasmid according to claim 4, wherein the codons are trinucleotide codons.

6. A plasmid according to any preceding claim, wherein the source of the plasmid vector is a microorganism selected from the genus Eschericia.

7. A plasmid according to claim 6, said vector comprising E.coli plasmid pBR322.

8. A transformant microorganism including a recombinant plasmid, said plasmid comprising a vector having inserted therein a synthetic production gene which codes for the expression of human insulin-like growth factor -I(IGF-I).

9. The transformant microorganism of claim 8, wherein the microorganism is selected from the genus Eschericia.

10. The transformant microorganism of claim 9, wherein the microorganism is E.coli.

11. The transformant microorganism of claim 10, wherein the microorganism is E.coli K12 strains JM 83 and 294.

12. A polydeoxyribonucleotide segment which codes for the expression of human insulin-like growth factor -I(IGF-I).

13. The polydeoxyribonucleotide segment according to claim 12 having restriction endonuclease cleavage sites at the termini thereof, a start codon (ATG) inside one of said sites and a stop codon (TAG) inside the other one of said sites, and a gene coding for the expression of human insulin-like growth factor -I(IGF-I) between said codons.

14. The polydeoxyribonucleotide segment according to claim 13 essentially as shown in Fig. 2.

15. A process for producing human insulin-like growth factor -I protein (IGF-I) which comprises culturing a transformant microorganism including a recombinant plasmid, said plasmid comprising a synthetic production gene which codes for the expression of human insulin-like growth factor -I(IGF-I), in a suitable nutrient medium until the protein is formed, and then isolating the said protein.

16. Methionyl -IGF-I.

17. A fused protein comprising methionyl-IGF-I or IGF-I.

TB/On

Fig-1

MET GLY PRO GLU THR LEU CYS GLY ALA GLU LEU VAL ASP ALA LEU GLN PHE VAL

ATG GGT CCC GAA ACT CTG TGC GGT GCT GAA CTG GTT GAC GCT CTG CAG TTT GTT

CYS GLY ASP ARG GLY PHE TYR PHE ASN LYS PRO THR GLY TYR GLY SER SER SER ARG ARG

TGC GGT GAC CGT GGT TTT TAT TTT AAC AAA CCC ACT GGT TAT GGT TCT TCT TCT CGT CGT

ALA PRO GLN THR GLY ILE VAL ASP GLU CYS CYS PHE ARG SER CYS ASP LEU ARG ARG LEU

GCT CCC CAG ACT GGT ATT GTT GAC GAA TGC TGC TTT CGT TCT TGC GAC CTG CGT CGT CTG

GLU MET TYR CYS ALA PRO LEU LYS PRO ALA LYS SER ALA

GAA ATG TAT TGC GCT CCC CTG AAA CCC GCT AAA TCT GCT

The IGF-1 protein and the selected DNA code.

The molecular weight of this protein is 7775.93.

```
    A1          A2                A3              A4                A5
5'GGAATTCT ATGGGTCCCGAAAC TCTGTGCGGTGCTG AACTGGTTGACGCT CTGCAGTTTGTTTG CGGTGA
3'CCTTAAGATACCCA GGGCTTTGAGACAC GCCACGACTTGACC AACTGCGAGACGTC AAACAAACGCCACT
      B17              B16              B15             B14              B13

A6           A7                A8              A9              A10            A11
CCGTGGTT TTTATTTTAACAAA CCCACTGGTTATGG TTCTTCTTCTCGTC GTGCTCCCCAGACT GGTATTG
GGCACCAAAAATAA  AATTGTTTGGGTGA CCAATACCAAGAAG AAGAGCAGCACGAG GGGTCTGACCATAA
    B12              B11              B10             B9               B8

               A12               A13             A14              A15
TTGACGA ATGCTGCTTTCGTT  CTTGCGACCTGCGT CGTCTGGAAATGTA TTGCGCTCCCCTGA AACCCG
CAACTGCTTACGAC GAAAGCAAGAACGC TGGACGCAGCAGAC CTTTACATAACGCG AGGGGACTTTGGGC
      B7              B6              B5              B4               B3

A16          A17
CTAAATCT GCTTAGAAGCTTGG3'
GATTTAGACGAATC TTCGAACC5'
      B2            B1
```

Fig-2

The two DNA-strands (for the IGF-1 gene) divided into oligomers A1-A17 and B1-B17.
Besides the DNA code described in Fig.1 the sequence has been provided with a start codon (ATG - in block A2), a stop codon (TAG - in block A17) and sequences for EcoRI (block A1) and HindIII (block A17).

```
ECO RI     AVA IIB                                    HGA IAPST I              BSTE IIC
                                                   HINC IIC                    HPH IA
GGAATTCTATGGGTCCCGAAACTCTGTGCGGTGCTGAACTGGTTGACGCTCTGCAGTTTGTTTGCGGTGACCGTGGTTTT
                   20                  40                  60                  80

                         MBO IIB      HGIA IC            HINC IIC  BBV IB
                            MBO IIB
TATTTTAACAAACCCACTGGTTATGGTTCTTCTTCTCGTCGTGCTCCCCAGACTGGTATTGTTGACGAATGCTGCTTTCG
                  100                 120                 140                 160

           HGA IB             HHA I                                 ALU I
                                                              DDE ID
                                                                   HIND III
TTCTTGCGACCTGCGTCGTCTGGAAATGTATTGCGCTCCCCTGAAACCCBCTAAATCTGCTTAGAAGCTTGG
                  180                 200                 220
```

Fig. 3

Fig. 4

A2

Si-C + A → Si-CA + A → Si-CAA + A → Si-CAAA + G →

Si-CAAAG + C → Si-CAAAGC + C → Si-CAAAGCC + C →

Si-CAAAGCCC + T → Si-CAAAGCCCT + G →

Si-CAAAGCCCTG + G → Si-CAAAGCCCTGG + G →

Si-CAAAGCCCTGGG + T → Si-CAAAGCCCTGGGT + A →

Si-CAAAGCCCTGGGTA

Fig. 5

DMTr=

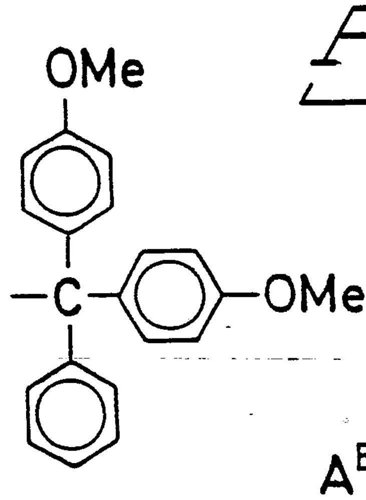

$A^{Bz}$=

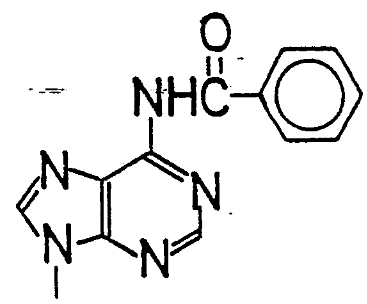

$C^{Bz}$ =

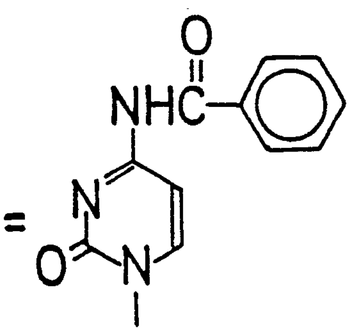

Fig-6

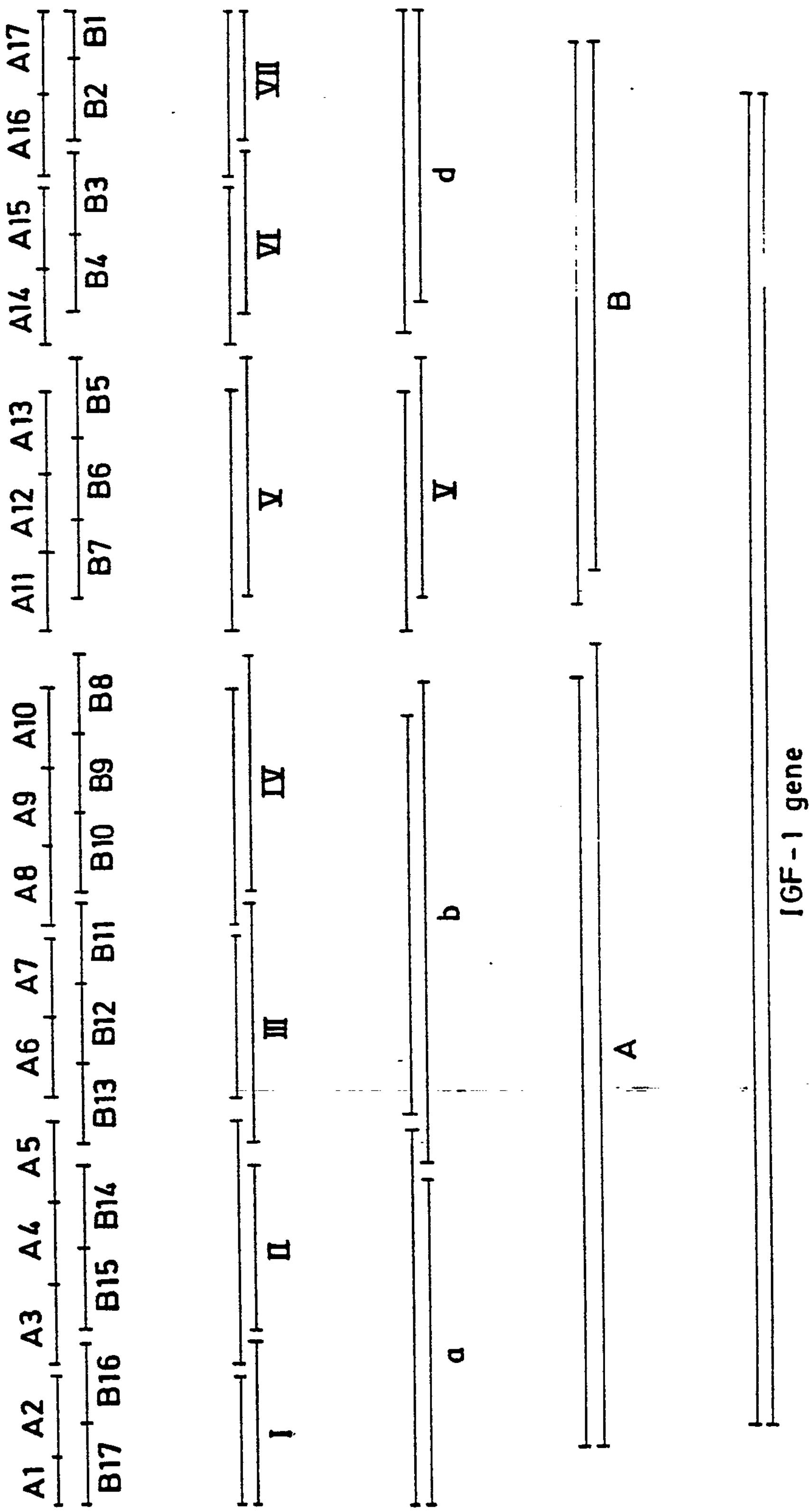


Ligation pattern for the IGF-1 gene

Fig. 7

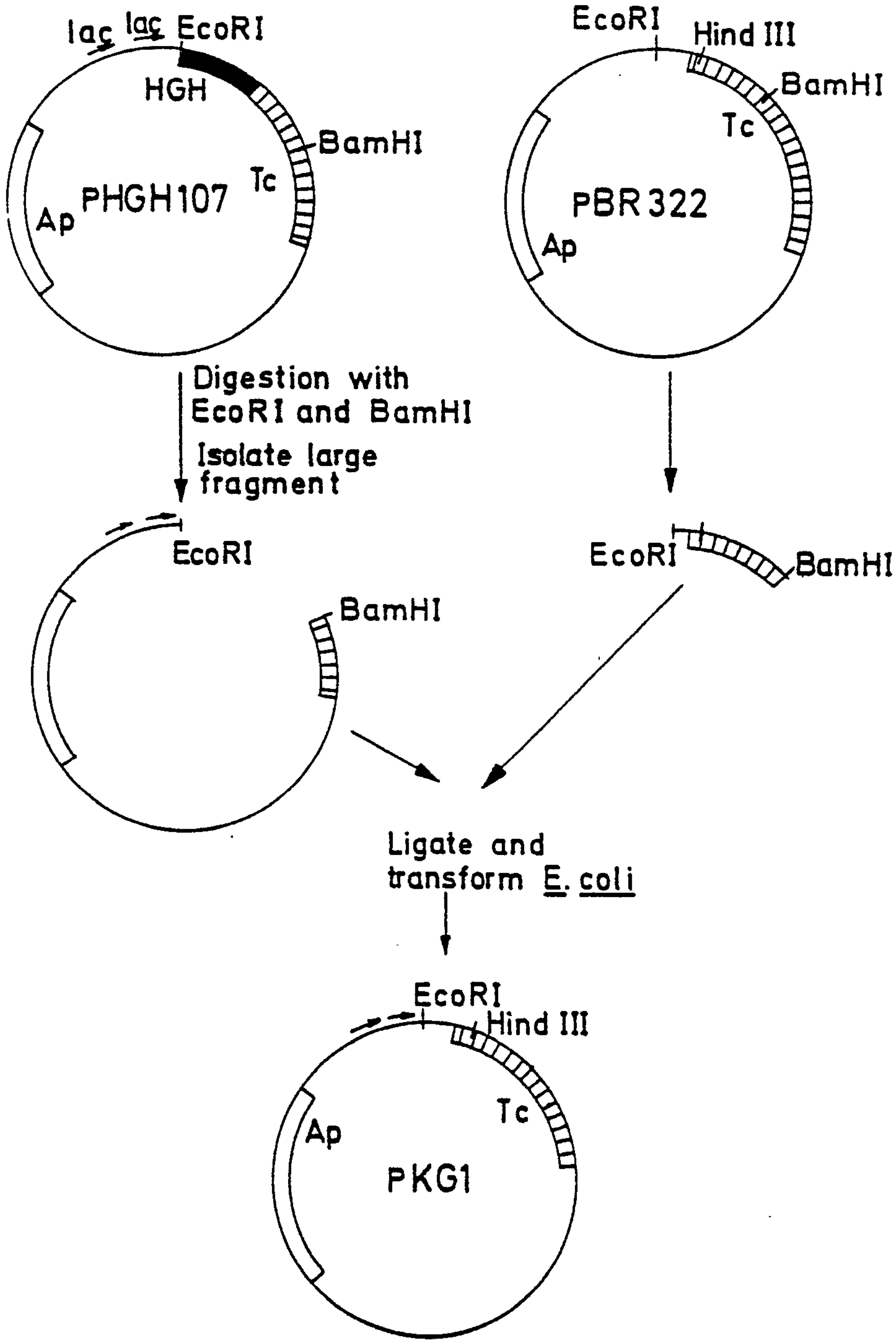
lac lac EcoRI
HGH
BamHI
Tc
Ap PHGH107
EcoRI
Hind III
BamHI
Tc
PBR322
Ap
Digestion with
EcoRI and BamHI
Isolate large
fragment
EcoRI
BamHI
EcoRI
BamHI
Ligate and
transform E. coli
EcoRI
Hind III
Tc
Ap
PKG1

Fig. 8

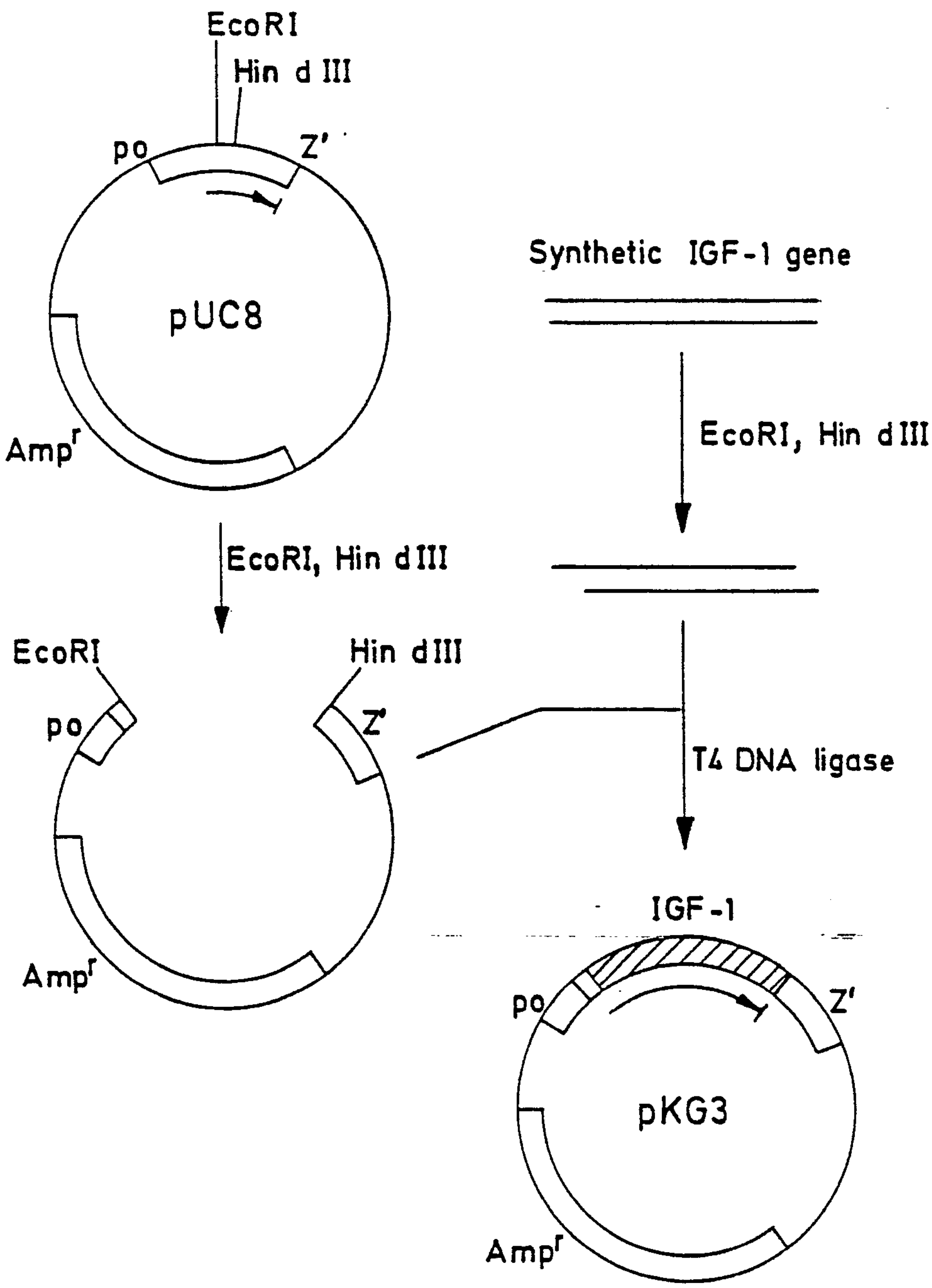
EcoRI
Hin d III
po
Z'
pUC8
Amp^r
Synthetic IGF-1 gene
EcoRI, Hin dIII
EcoRI, Hin dIII
EcoRI
Hin dIII
po
Z'
Amp^r
T4 DNA ligase
IGF-1
po
Z'
pKG3
Amp^r

Fig. 9

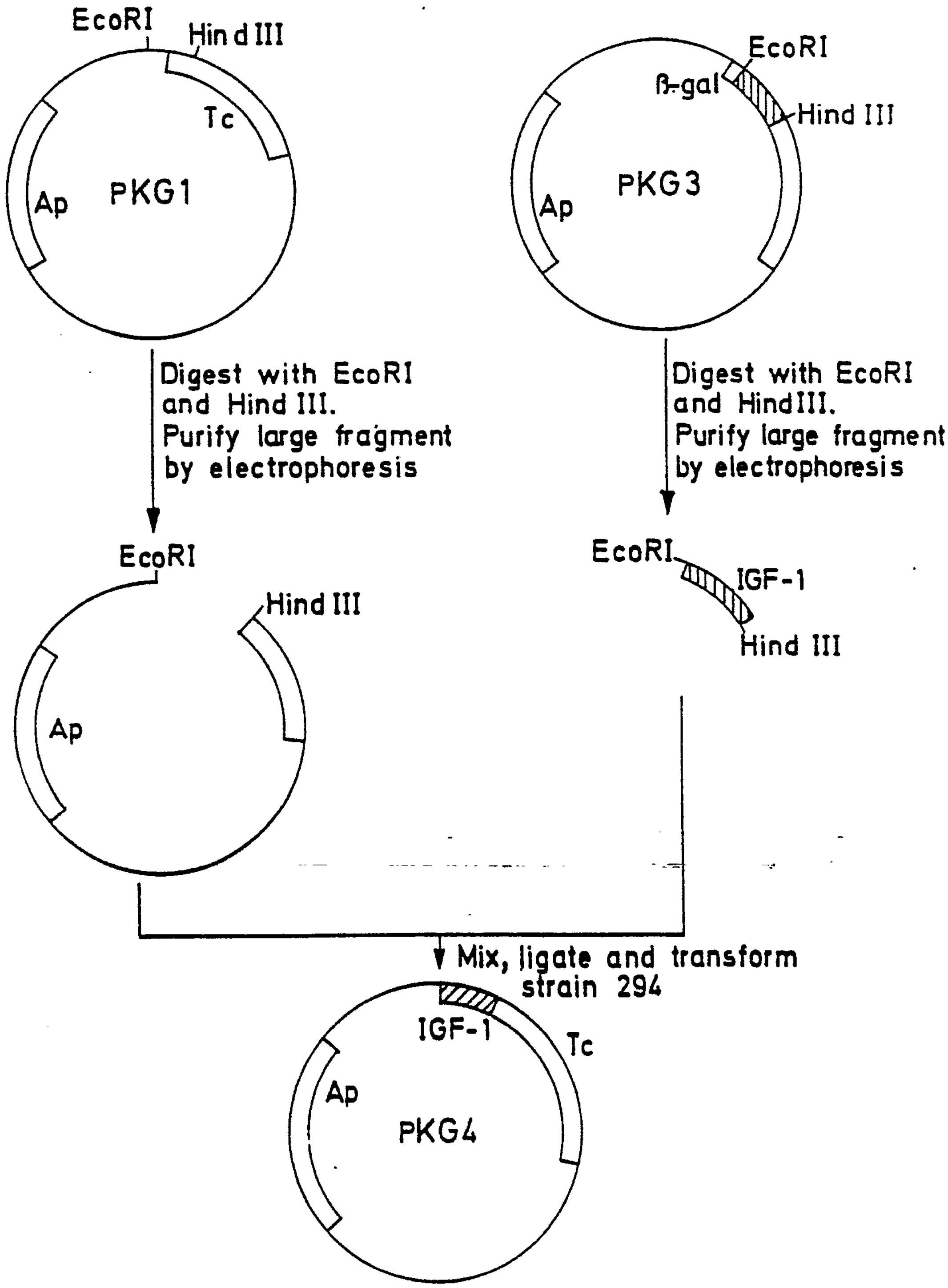
EcoRI
Hin d III
Tc
Ap
PKG1
EcoRI
β-gal
Hind III
Ap
PKG3
Digest with EcoRI
and Hind III.
Purify large fragment
by electrophoresis
Digest with EcoRI
and HindIII.
Purify large fragment
by electrophoresis
EcoRI
Hind III
Ap
EcoRI
IGF-1
Hind III
Mix, ligate and transform
strain 294
IGF-1
Tc
Ap
PKG4

Fig-10

| Sequence | | Cleavage | Plasmid |
|---|---|---|---|
| EcoR1<br>G\|AAT TCT ATG GGT CCC<br>Met Gly Pro | IGF-1 | ---- | pKG3 |
| G AAT TCT ATG GAC CCC<br>Met Asp↑Pro | IGF-1Asp | Formic acid | pKG11 pUN201 |
| G AAT TCT AAC GGT CCC<br>Asn↑Gly Pro | IGF-1 Asn | Hydroxylamine | pKG15 pUN204 |
| G AAT TCT TGG GGT CCC<br>Trp↑Gly Pro | IGF-1 Trp | NBS | pKG16 pUN207 |

Fig. 11

IGF-1 Asp

3'-......CCGGTCACTTAAGATACCCAGGGCTTTGAGACACGCCAC.....-5'
**
5'-GTGAATTCTATGGACCCCGAAACT-3'
ValAsnSerMetAspProGluThr

IGF-1 Asn

3'-......CCGGTCACTTAAGATACCCAGGGCTTTGAGACACGCCAC.....-5'
**
5'-TGAATTCTAACGGTCCCGAA-3'
ValAsnSerAsnGlyProGlu

IGF-1 Trp

3'-......CCGGTCACTTAAGATACCCAGGGCTTTGAGACACGCCAC.....-5'
**
5'-GTGAATTCTTGGGGTCCCGA-3'
ValAsnSerTrpGlyProGlu

Fig. 19

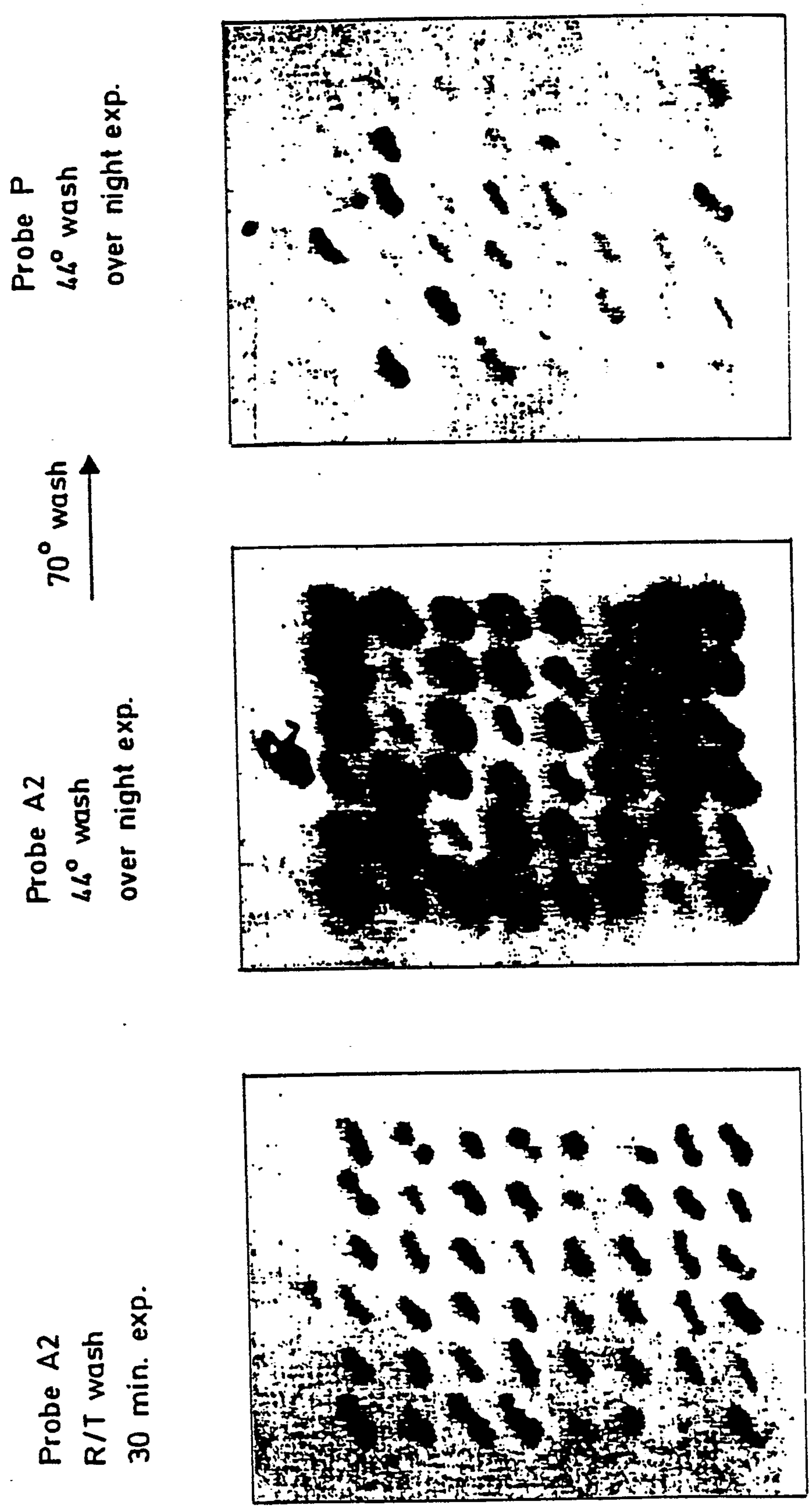
Probe A2
R/T wash
30 min. exp.
Probe A2
44° wash
over night exp.
70° wash
Probe P
44° wash
over night exp.

Fig. 13

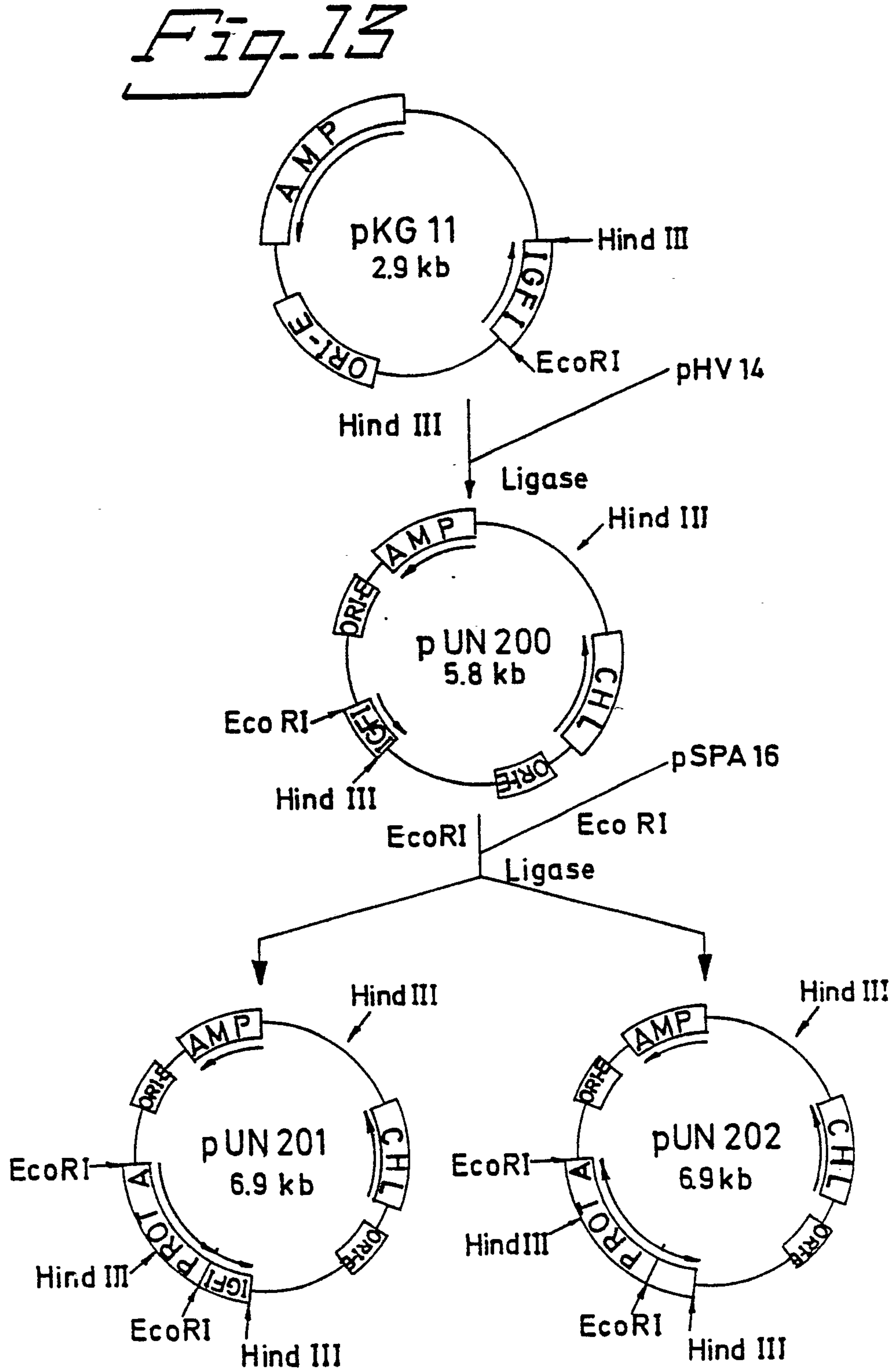
AMP
pKG 11
2.9 kb
Hind III
IGF I
ORI-E
EcoRI
pHV 14
Hind III
Ligase
Hind III
AMP
ORI-E
p UN 200
5.8 kb
CHL
Eco RI
IGF I
ORI-E
Hind III
pSPA 16
EcoRI
Eco RI
Ligase
Hind III
AMP
ORI-E
p UN 201
6.9 kb
CHL
EcoRI
PROT A
ORI-E
Hind III
IGF I
EcoRI
Hind III
Hind III
AMP
ORI-E
pUN 202
6.9 kb
CHL
EcoRI
PROT A
HindIII
ORI-E
EcoRI
Hind III

# Fig. 14

GCGCAACACGATGAAGCTCAACAAAATGCTTTTTATCAAGTCTTAAATATGCCTAACTTAAATGCTGATCAA 72
AlaGlnHisAspGluAlaGlnGlnAsnAlaPheTyrGlnValLeuAsnMetProAsnLeuAsnAlaAspGln

CGCAATGGTTTTATCCAAAGCCTTAAAGATGATCCAAGCCAAAGTGCTAACGTTTTAGGTGAAGCTCAAAAA 144
ArgAsnGlyPheIleGlnSerLeuLysAspAspProSerGlnSerAlaAsnValLeuGlyGluAlaGlnLys

CTTAATGACTCTCAAGCTCCAAAAGCTGATGCGCAACAAAATAACTTCAACAAAGATCAACAAAGCGCCTTC 216
LeuAsnAspSerGlnAlaProLysAlaAspAlaGlnGlnAsnAsnPheAsnLysAspGlnGlnSerAlaPhe

TATGAAATCTTGAACATGCCTAACTTAAACGAAGCGCAACGTAACGGCTTCATTCAAAGTCTTAAAGACGAC 288
TyrGluIleLeuAsnMetProAsnLeuAsnGluAlaGlnArgAsnGlyPheIleGlnSerLeuLysAspAsp

CCAAGCCAAAGCACTAACGTTTTAGGTGAAGCTAAAAAATTAAACGAATCTCAAGCACCGAAAGCTGATAAC 360
ProSerGlnSerThrAsnValLeuGlyGluAlaLysLysLeuAsnGluSerGlnAlaProLysAlaAspAsn

AATTTCAACAAAGAACAACAAAATGCTTTCTATGAAATCTTGAATATGCCTAACTTAAACGAAGAACAACGC 432
AsnPheAsnLysGluGlnGlnAsnAlaPheTyrGluIleLeuAsnMetProAsnLeuAsnGluGluGlnArg

AATGGTTTCATCCAAAGCTTAAAAGATGACCCAAGCCAAAGTGCTAACCTATTGTCAGAAGCTAAAAAGTTA 504
AsnGlyPheIleGlnSerLeuLysAspAspProSerGlnSerAlaAsnLeuLeuSerGluAlaLysLysLeu

AATGAATCTCAAGCACCGAAAGCGGATAACAAATTCAACAAAGAACAACAAAATGCTTTCTATGAAATCTTA 576
AsnGluSerGlnAlaProLysAlaAspAsnLysPheAsnLysGluGlnGlnAsnAlaPheTyrGluIleLeu

Fig. 14 CONTD.

CATTTACCTAACTTAAACGAAGAACAACGCAATGGTTTCATCCAAAGCCTAAAAGATGACCCAAGCCAAAGC 648
HisLeuProAsnLeuAsnGluGluGlnArgAsnGlyPheIleGlnSerLeuLysAspAspProSerGlnSer

GCTAACCTTTTAGCAGAAGCTAAAAAGCTAAATGATGCTCAAGCACCAAAAGCTGACAACAAATTCAACAAA 720
AlaAsnLeuLeuAlaGluAlaLysLysLeuAsnAspAlaGlnAlaProLysAlaAspAsnLysPheAsnLys

GAACAACAAAATGCTTTCTATGAAATTTTACATTTACCTAACTTAACTGAAGAACAACGTAACGGCTTCATC 792
GluGlnGlnAsnAlaPheTyrGluIleLeuHisLeuProAsnLeuThrGluGluGlnArgAsnGlyPheIle

EcoR1
CAAAGCCTTAAAGACGATCCGGGGAATTCTATGGATCCCGAAACTCTGTGCGGTGCTGAACTGGTTGACGCT 864
GlnSerLeuLysAspAspProGlyAsnSerMetAspProGluThrLeuCysGlyAlaGluLeuValAspAla

CTGCAGTTTGTTTGCGGTGACCGTGGTTTTTATTTTAACAAACCCACTGGTTATGGTTCTTCTTCTCGTCGT 936
LeuGlnPheValCysGlyAspArgGlyPheTyrPheAsnLysProThrGlyTyrGlySerSerSerArgArg

GCTCCCCAGACTGGTATTGTTGACGAATGCTGCTTTCGTTCTTGCGACCTGCGTCGTCTGGAAATGTATTGC 1008
AlaProGlnThrGlyIleValAspGluCysCysPheArgSerCysAspLeuArgArgLeuGluMetTyrCys

HindIII
GCTCCCCTGAAACCCGCTAAATCTGCTTAGAAGCTT 1044
AlaProLeuLysProAlaLysSerAla ***

European Patent Office

# EUROPEAN SEARCH REPORT

0130166
Application number

EP 84 85 0197

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | THE JOURNAL OF BIOLOGICAL CHEMISTRY, vol. 253, no. 8, April 1978, pages 2769-2776(USA); E. RINDERKNECHT, R.E.HUMBEL: "The Amino Acid Sequence of Human Insulin-like Growth Factor I and Its Structural Homology with Proinsulin". *Fig. 1, page 2771* | 1-17 | C 12 N 15/00 |
| Y | EP-A-0 075 444 (GENENTECH, INC.) *Claims 1,3,7-13; fig. 4 * | 1-11,15 | |
| | | | TECHNICAL FIELDS SEARCHED (Int. Cl. 3) |
| | | | C 12 N |

The present search report has been drawn up for all claims

| Place of search | Date of completion of the search | Examiner |
|---|---|---|
| STOCKHOLM | 08-08-1984 | PETERSSON I-K |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document